(19) Europäisches Patentamt
European Patent Office
Office européen des brevets

(11) EP 1 003 458 B1

(12) **EUROPEAN PATENT SPECIFICATION**

(45) Date of publication and mention of the grant of the patent:
**23.10.2002 Bulletin 2002/43**

(21) Application number: **98936568.9**

(22) Date of filing: **03.08.1998**

(51) Int Cl.[7]: **A61B 5/00**, A61K 49/00, G01N 33/48

(86) International application number:
**PCT/GB98/02322**

(87) International publication number:
**WO 99/007325 (18.02.1999 Gazette 1999/07)**

(54) **LIVER FUNCTION TEST**

LEBERFUNKTIONSTEST

TEST HEPATIQUE

(84) Designated Contracting States:
**AT BE CH CY DE DK ES FI FR GB GR IE IT LI LU MC NL PT SE**
Designated Extension States:
**AL LT LV MK RO SI**

(30) Priority: **12.08.1997 GB 9716962**

(43) Date of publication of application:
**31.05.2000 Bulletin 2000/22**

(73) Proprietor: **Norgine Europe BV
1011 CA Amsterdan (NL)**

(72) Inventor: **MILLS, Charles Oswald
Kings Heath, Birmingham B14 7AJ (GB)**

(74) Representative: **Pearce, Anthony Richmond
MARKS & CLERK,
Alpha Tower,
Suffolk Street Queensway
Birmingham B1 1TT (GB)**

(56) References cited:
**WO-A-97/06829**
**US-A- 4 264 514**
**US-A- 4 848 349**

- **C.O.MILLS ET AL.: "Cholyllysyl Fluorescein and Related Lysyl Fluorescein Conjugated Bile Acid Analogues" YALE J. BIOL. MED., vol. 70, no. 4, 1997, pages 447-457, XP002085635 UK cited in the application**
- **DATABASE CHEMABS CHEMICAL ABSTRACTS SERVICE, COLUMBUS, OHIO, US MILLS, C. O. ET AL: "Cholyl-lysylfluorescein: synthesis, biliary excretion in vivo and during single-pass perfusion of isolated perfused rat liver" XP002082298 & BIOCHIM. BIOPHYS. ACTA (1991), 1115(2), 151-6 CODEN: BBACAQ;ISSN: 0006-3002,**
- **BAXTER D J ET AL: "BILIARY LIPID OUTPUT BY ISOLATED PERFUSED RAT LIVERS IN RESPONSE TOCHOLYL-LYSYLFLUORESCEIN" BBA - LIPIDS AND LIPID METABOLISM, vol. 1256, no. 3, 1995, pages 374-380, XP000615216**

EP 1 003 458 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to a method for the determination of liver function (i.e. to enable identification of the nature of a liver disease in a patient to be determined).

Uptake and biliary secretion of bile acids are important hepatic functions and an elevated concentration of serum bile acids is observed in several parenchymal and cholestatic liver diseases. Rapid clearance of natural [Korman et. al. New Eng. J. Med. (1975) **292,** 1205] and radiolabelled [Horak et. al. Gastroenterology (1976) **71,** 809] bile acids from plasma with a good correlation between impaired plasma clearance and hepatic dysfunction in patients with different liver diseases has been shown. P.-H. Ekdahl et al [Atomlight (1966), No. 56, 1-5 Coden, ATLGA4, XP002063328] disclose the use of $C^{14}$ labelled cholic acid in a liver function test in which the capacity to conjugate cholic acid is assessed using homogenized human liver samples. However all of these methods entail complicated procedures and, in the latter two cases, the necessity of using radioactive compounds.

J. M. Crawford et al [Biochim. Biophys. Acta, 1991, 1085(2), 223-34] disclose the use of fluorescent dansylated bile salt derivatives in the determination of the influence of the hydroxy groups in the steroid ring on hepatocellular transport and biliary excretion.

US 4848349 discloses fluorescently labelled bile acids for diagnostic and research use. One of the uses described is for indirect measurement of hepatic blood flow, such use involving sampling blood over a period of time and analysing the elimination rate of the labelled bile acid from the plasma so as to give an indication of the hepatic blood flow.

US 4264514 discloses an assay for determining the level of conjugated bile acid in a serum sample by mixing the serum sample with (a) an antiserum specific to one of the conjugated bile acids in the sample, and (b) a reagent containing the same conjugated bile acid which has been labelled or tagged so as to bind the antibodies of the antiserum to the specific conjugated bile acid in the sample and to the labelled conjugated bile acid; separating the antibody-bound material from the antibody-unbound material, and then measuring the level of the labelled derivative in either of the separated fractions so as to enable the quantity of the specific bile acid conjugate in the sample to be determined by comparison with standards. The possibility of using fluorescent chemicals, radioactive isotopes or electron spin-resonating chemicals for labelling or tagging the conjugated bile acid derivative is disclosed. However, this procedure is relatively expensive and complicated since it involves the use of both a specific antiserum and a specific labelled conjugated bile acid for each conjugated bile acid being determined in the sample.

C.O. Mills et al in Biochimica et Biophysica Acta, 1115 (1991), 151-156 relates to a study of the fluorescent bile salt, cholyl-lysylfluorescein, in which the biliary kinetics and hepatic extraction are studied in vivo and in vitro in rats. The similarity in biliary output and hepatic extraction of cholyl-lysylfluorescein to that of the natural bile acid cholylglycine is noted and it is suggested that this material may allow non-invasive estimation of liver blood flow by blood sampling in patients with liver disease. It is also suggested from the studies that an appropriate bile acid may be used to target a drug to the liver whilst retaining its physicochemical properties.

It is an object of the present invention to provide a method of determining the liver function in which the above disadvantages can be obviated or mitigated.

According to the present invention there is provided an in-vitro method of determining the liver function of a patient, comprising the steps of:-

(i) processing (utilising) a series of blood samples which have passed through the liver of the patient and which have been collected at timed intervals after introducing an effective amount of a coloured or fluorescent bile acid derivative intravenously into the patient; and

(ii) assessing the colour or fluorescence of bile acid derivative in each sample by:-

  (a) processing the blood sample to obtain blood plasma containing the bile acid derivative,

  (b) measuring the colour or fluorescence of the bile acid derivative in the blood plasma, and

  (c) fitting the measurements obtained in step (b) to a plasma elimination curve and comparing the plasma elimination curve with plasma elimination curves obtained from individuals having known liver function, to arrive at a determination of liver function for the patient.

The present invention also resides in the use of a coloured or fluorescent bile acid derivative in the manufacture of a diagnostic agent for the determination of liver function by a method which comprises the steps of:-

(i) introducing an effective amount of the diagnostic agent containing the coloured or fluorescent bile acid derivative intravenously into a patient,

(ii) collecting samples of blood which has passed through the liver of the patient at timed intervals after step (i), and

(iii) assessing the colour or fluorescence of bile acid derivative in each sample by:-

(a) processing the blood sample to obtain blood plasma containing the bile acid derivative,
(b) measuring the colour or fluorescence of the bile acid derivative in the blood plasma, and
(c) fitting the measurements obtained in step (iii)(b) to a plasma elimination curve and comparing the plasma elimination curve with plasma elimination curves obtained from individuals having known liver function, to arrive at a determination of liver function for the patient.

The bile acid derivative preferably comprises a steroid moiety having an unblocked 3-hydroxyl, 7-hydroxyl or 12-hydroxyl substituent or any combination thereof, and an unblocked carboxyl group attached by means of an amide linkage to the side chain of the steroid moiety, and an active moiety which is to be targeted to the liver, said active moiety being attached to the α-carbon atom relative to the unblocked carboxyl group.
Preferably, said compound has the general formula (I):-

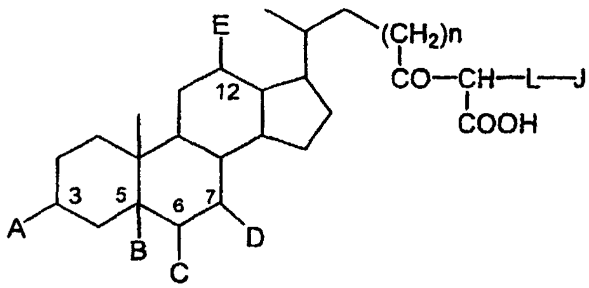

(I)

wherein A is α-OH or β-OH; B is α-H or β-H; C is -H, α-OH or β-OH; or B and C together form a double bond; D is -H, α-OH or β-OH; E is -H, α-OH or β-OH; L is a linking moiety; J is said coloured or fluorescent moiety; and n is 0 or 1.
Preferably, J is or includes a fluorescein, rhodamine or other fluorescing moiety.
The linking moiety is preferably N-terminated at its end attached to active moiety J, and L may be:-

$$-(CH_2)_nNH,$$

where n is 3 or 4,

$$-(CH_2)_4NH-(CH_2)_3NHC(=NH)NH-,$$

or

$$-(CH_2)_2-CH(OH)CH_2NH-.$$

Alternatively, the moiety -NH-CH(COOH)-L- may be derived from S-adenosylhomocysteine, S-adenosylmethionine, S-amino-imadazole-4-carboxamide, asparagine, cadaverine, cystamine, citrulline, diaminopimelic acid, 2,4-diaminobutyric acid, cysteamine, glutamine, 3-hydroxykynurenine, kynurenine, putrescine or negamycin. Alternatively, acidic amino acids can be used instead of the above where active moiety J has one amino group and/or is hydrophobic.
Preferably, the steroid moiety of the bile acid derivative is based on cholic acid, chenodeoxycholic acid, deoxycholic acid, hyodeoxycholic acid, hyocholic acid, α-, β- or ω-muricholic acid, nor-bile acids, lithocholic acid, 3β-hydroxycholenoic acid, ursodeoxycholic acid, allocholic acid (5α-cholan-24-oic-acid), or the like.
Most preferably, the fluorescent bile acid derivative is a cholyl-lysylfluorescein (CLF) having the formula:-

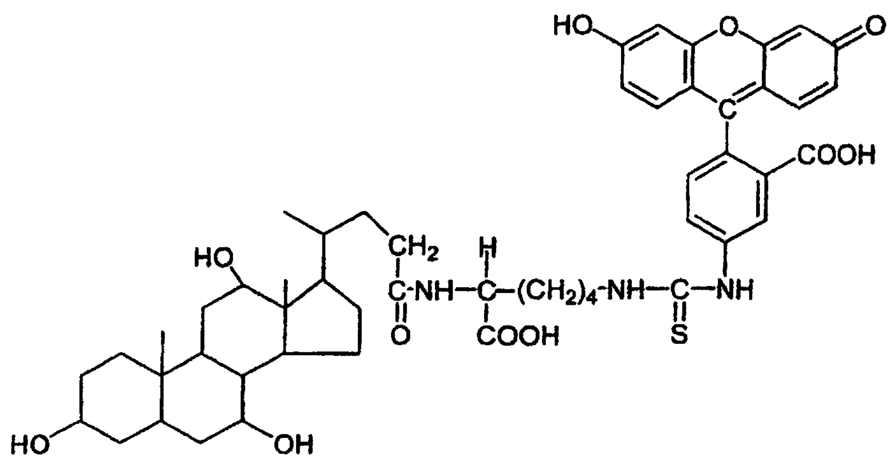

wherein the lysyl and fluorescein are linked by a thioamido moiety -C(S)NH-. This compound has in the past been referred to simply as "CLF", and "CLF" as used hereinafter is to be construed in this way.

In animal models, lysyl-fluorescein analogues of bile acids present several physical and physiological properties, including hepatic uptake [Saraswat et. al. Gut (1995) **36** (Suppl. 1), A18], which closely resemble those of natural bile acids.

Step (a) of the method may be achieved by centrifugation of the sample.

Preferably, for a fluorescent bile acid derivative, step (a) additionally comprises separation of blood proteins from the plasma, for example by the addition of methanol to the plasma to precipitate the proteins, followed by centrifugation. If the blood proteins are not separated, they can complex with the fluorescent bile acid derivative and quench fluorescence, thereby giving artificially low and variable fluorescence measurements.

It may be desirable to include an additional step of diluting the plasma between steps (a) and (b).

Preferably, the coloured or fluorescent bile acid derivative is injected as a saline solution, the volume of said solution preferably being in the range of 1 $cm^3$ to 10 $cm^3$.

The dose is preferably at least 0.02 mg/kg b.w. and preferably in the range of 0.02 to 0.5 mg/kg b.w.

It has been found that serum fluorescence in samples taken at 30 minutes after injection of CLF show the highest discriminative value between a group of people with healthy livers, a group of patients suffering from liver cirrhosis and a group of patients with fatty livers. Accordingly, it may be particularly convenient to assess a sample taken at about 30 minutes after injection and to compare the serum fluorescence value obtained with at least one standard to obtain an indication of liver function.

Thus, in a further aspect of the present invention, there is provided a method of determining the liver function of a patient,
comprising the steps of:-

(i) processing (utilising) a blood sample which has passed through the liver of the patient and which has been collected a predetermined time interval after introducing an effective amount of a coloured or fluorescent bile acid derivative intravenously into the patient; and

(ii) assessing the colour or fluorescence of bile acid derivative in the sample by:-

(a) processing the blood sample to obtain blood plasma containing the bile acid derivative,

(b) measuring the colour or fluorescence of the bile acid derivative in the blood plasma, and

(c) comparing the measurement obtained in step (b) with at least one standard so as to arrive at a determination of liver function for the patient.

Apart from step (c), the procedures involved may be equivalent to the procedures utilised in the method involving the processing and assessment of a plurality of samples collected at timed intervals after administration.

An embodiment of the invention will now be described by way of example with reference to the drawings, in which:-

Fig. 1 shows a plasma elimination curve for cholyl-lysyl-fluorescein (CLF) in healthy volunteers, and
Fig. 2 shows CLF plasma clearance curves for healthy patients and patients with different causes of liver dysfunction.

CLF was synthesized as described elsewhere [Mills et. al. Biochim. Biophys. Acta (1991) **1115**, 151], sterilized and tested for the presence of pyrogens.

A study was carried out in six healthy volunteers (1 female, 5 male), ages 30-53 years. Subjects were studied supine after overnight fasting. After an initial basal blood sample was taken, CLF in a dose of 0.02 mg/kg of body weight (b.w.) in physiological saline was injected intravenously during 15 seconds. Venous blood samples were collected from the opposite antecubital vein into gel and lithium heparin containers (sold under the Trade Name Vacutainers by Becton Dickinson Vacutainer Systems) at 2, 3, 4, 6, 10, 15, 20, 30, 60 and 90 minutes. The blood samples were then centrifuged and 0.5 ml aliquots of plasma were taken and added to 3.5 ml of methanol to precipitate plasma proteins, followed by further centrifugation. The supernatant (1 ml) was diluted to 3 ml with methanol and the fluorescence was then measured by spectrometer (Perkin Elmer LS5B Luminescence Spectrometer).

CLF concentration (μg/ml) was derived from a calibration curve which expressed the equation:

$$y = 2566x - 52.6$$

where y represents the measured fluorescence in fluorescence units at an excitation wavelength of 490 nm with emission at 520 nm and x represents the CLF concentration in μg/ml. The calibration curve was calibrated from fluorescence measurements of solutions having known CLF concentrations followed by linear regression analysis.

Non linear plasma elimination curves presented as percentage dose administered per litre of plasma were fitted to a tri-exponential model. The half life ($t_{½}$ time) was calculated for the first ($t_{½\ 1st\ phase}$), second ($t_{½\ 2nd\ phase}$) and third ($t_{½\ 3rd\ phase}$) phases of the elimination curves from the equations:

$$t_{½\ 1st\ phase} = 0.693/k_1 \qquad t_{½\ 2nd\ phase} = 0.693 / k_2 \qquad t_{½\ 3rd\ phase} = 0.693 / k_3$$

where $k_1$, $k_2$ and $k_3$ were calculated for each subject using the equation:

$$C_t = Ae^{-k_1 t} + Be^{-k_2 t} + Ce^{-k_3 t}$$

in which:

$C_t$ is the concentration of CLF at time t,
A, B and C are the intercepts of each phase of elimination at t=0
$k_1$, $k_2$ and $k_3$ are fractional disappearance rates of the three phases of elimination.

The resulting plasma elimination curve is shown in Fig 1.

There is extensive experience of exposure to the active moieties of CLF (cholyl-glycine and fluorescein) in humans. Cholyl-glycine is a naturally occurring primary bile salt in humans and intravenous fluorescein angiography is commonly performed in ophthalmology. Advantageously, the present invention allows the use of extremely small quantities of CLF. By comparison, the dose of glycocholate routinely employed in intravenous preparations such as Vit $K_1$ or Diazepam is approximately 300 times greater than the amount of CLF which may be used in the present invention and the dose of fluorescein applied in fundus fluorescein angiography is 400 times greater.

The plasma elimination curve for CLF showed three phases of elimination. $t_{½}$ for the first, second and third phases of elimination were 1.7±0.9 min, 6.7±1.6 min and 68±17 min respectively. Data for radiolabelled cholyl glycine reported by Cowen et. al. [Gastroenterology (1975) **68,** 1567] gave $t_{½}$ for the first and the second exponentials as 1.7±0.1 and 7±0.1 min respectively. The cholyl glycine data were fitted to an elimination curve based on a bi-exponential model, but it is now thought to be more appropriate to apply a tri-exponential analysis [Engelking et. al. Clinical Science (1979) **57**, 499]. Plasma retention of CLF at 90 minutes, when expressed as a percentage of the dose administered per litre of plasma was 2.2%.

In five healthy volunteers, a 25-fold higher dose of CLF (0.5 mg/kg b.w.) was injected in order to further assess the appropriateness of the dose chosen and the safety of the compound. Serum biochemistry including basic liver function tests, electrolytes and kidney function did not change significantly following this injection. Table. 1 shows values of standard liver function tests and plasma concentration of urea and creatinine before and after injection of the

higher dose of CLF. Although there was a consistent diminution in plasma alkaline phosphatase levels post injection of CLF, this difference was not statistically significant (p=0.06).

Table 1.

| Tests of Liver Function Before And After CLF Dose of 0.5 mg/Kg b.w. For 5 Healthy Patients. | | | |
|---|---|---|---|
| test | before CLF injection | after CLF injection | difference |
| bilirubin (μM/L) | 10 | 11 | NS[3] |
| AST[1] (U/L) | 17 | 16 | NS |
| AlkP[2] (U/L) | 148 | 134 | p = 0.06 |
| Albumin (g/L) | 45 | 42 | NS |
| Urea (mM/L) | 4.4 | 4.3 | NS |
| Creatinine (μM/L) | 94 | 89 | NS |

1: AST = aspartate aminotransferase

2: AlkP =alkaline phosphatase

3: NS = not significant

Elimination curves obtained with the 25-fold increased dose confirmed that efficiency of hepatic clearance is unchanged. This reflects the behaviour of natural bile acids observed by Korman et. al. (referred to above). However, doses of CLF used in the present invention are much lower than the doses of natural bile acid utilized by Korman et. al. (0.02 - 0.5 mg/kg b.w. versus 5 mM/kg b.w. which equates to ≈2.4g/Kg b.w.). The volume of distribution of CLF calculated as proposed by Thiordleifsson et. al. [Gut (1977) **18**, 697] at 2214±147 ml, was similar to values for radiolabelled cholyl glycine reported by others [Gilmore et. al. Gut (1978) **19**, 1110].

The above results were also calculated in terms of the fluorescence at 60 min compared with the fluorescence at 10 min, expressed as a percentage. For radiolabelled cholic acid (Horak et. al., referred to above), this gave 60 min plasma retention of 8±1% for healthy volunteers compared to 71% for patients with fulminant hepatic failure (FHF) who subsequently died and 60% for FHF patients who survived (the difference between the two groups being statistically significant). The percentage of fluorescence after 60 min of 13.1±1 .6% for the present invention was similar to the data obtained for the healthy volunteers in Horak's work. The body of data showing that CLF plasma elimination is very similar to the elimination of natural bile acids suggests that there will be significant differences in plasma clearance of CLF between healthy individuals and those with liver dysfunction. This is confirmed by the data shown in Fig. 2. The elimination curves of Fig. 2 show differing rates of CLF elimination for (i) healthy patients, and patients with (ii) fatty, (iii) cirrhotic and (iv) recently transplanted livers (5 days post operation). Thus, not only does the present invention offer a useful means for determining liver function in a patient, it may also enable the cause of any dysfuncion to be diagnosed.

In a further study, CLF in the dose of 0.02 mg/kg b.w. was administered intravenously in 45 subjects: patients with liver cirrhosis(n=24), fatty liver (n = 12) and 9 healthy volunteers. Blood samples were collected before injection and every 10 minutes over 60 minutes. Plasma fluorescence was measured by luminescence spectrometer and residual fluorescence over the time of the study was compared in each group. Routine liver function tests (rLFT's) were performed before each injection.

It was observed that plasma clearance of CLF was significantly impaired in the patients with cirrhosis and fatty liver compared to the healthy subjects. Serum fluorescence at 30 minutes after injection of CLF($CLF_{30min}$) had the highest discriminative value between analysed groups (cirrhosis vs. fatty liver - p<0.0001; cirrhosis vs. healthy control - p<0.0001; fatty liver vs. healthy control - p<0.0025). $CLF_{30min}$ was more sensitive than routinely performed LFT's in detecting mild abnormalities of liver function in patients with fatty livers. No adverse reaction or side effects were observed.

The CLF test distinguished clearly between the three groups analysed and was more sensitive than rLFT's in detecting mild abnormalities of liver function. The test is safe and simple to perform and analyse and is therefore a potentially useful and convenient dynamic test of liver function.

## Claims

**1.** An in-vitro method of determining the liver function of a patient, comprising the steps of

(i) processing a series of blood samples which have passed through the liver of the patient and which have been collected at timed intervals after introducing an effective amount of a coloured or fluorescent bile acid derivative intravenously into the patient; and
(ii) assessing the colour or fluorescence of bile acid derivative in each sample by:-

(a) processing the blood sample to obtain blood plasma containing the bile acid derivative,
(b) measuring the colour or fluorescence of the bile acid derivative in the blood plasma, and
(c) fitting the measurements obtained in step (b) to a plasma elimination curve and comparing the plasma elimination curve with plasma elimination curves obtained from individuals having known liver function, to arrive at a determination of liver function for the patient.

**2.** A method as claimed in claim 1, wherein step (ii)(a) is achieved by centrifugation of the sample.

**3.** A method as claimed in claim 2, wherein a fluorescent bile acid derivative is employed, and step (ii)(a) additionally comprises separation of blood proteins from the plasma before centrifugation.

**4.** A method as claimed in claim 1, 2 or 3, including the additional step of diluting the plasma between steps (ii)(a) and (ii)(b).

**5.** A method as claimed in any preceding claim, wherein the bile acid derivative comprises (a) a steroid moiety having (i) at least one unblocked substituent selected from the group consisting of an unblocked 3-hydroxyl substituent, an unblocked 7-hydroxyl substituent and an unblocked12-hydroxyl substituent, and (ii) an unblocked carboxyl group attached by means of an amide linkage to a side chain of the steroid moiety; and (b) an active moiety which is to be targeted to the liver, said active moiety being attached to an α-carbon atom relative to the unblocked carboxyl group.

**6.** A method as claimed in any one of claims 1 to 4, wherein the bile acid derivative has the general formula (I):-

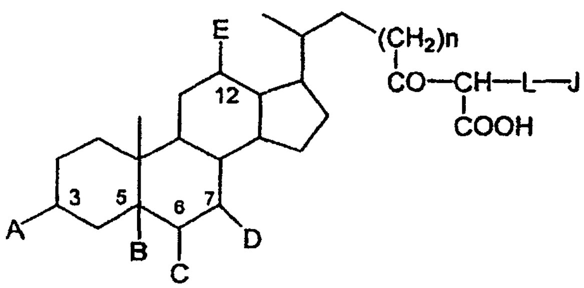

(I)

wherein A is selected from the group consisting of α-OH and β-OH; B is selected from the group consisting of α-H and β-H; C is selected from the group consisting of -H, α-OH and β-OH; or B and C together form a double bond; D is selected from the group consisting of -H, α-OH and β-OH; E is selected from the group consisting of -H, α-OH and β-OH; L is a linking moiety; J is said coloured or fluorescent moiety; and n is 0 or 1.

**7.** A method as claimed in claim 6, wherein J is or includes a fluorescein, rhodamine or other fluorescing moiety.

**8.** A method as claimed in claim 6 or 7, wherein the linking moiety is N-terminated at its end attached to active moiety J, and L is selected from the group consisting of:

$$-(CH_2)_nNH,$$

where n is 3 or 4,

$$-(CH_2)_4NH-(CH_2)_3NHC(=NH)NH-,$$

and

$$-(CH_2)_2-CH(OH)CH_2NH-.$$

**9.** A method as claimed in claim 6, wherein the moiety -NH-CH(COOH)-L- in the general formula (I) is derived from a compound selected from the group consisting of S-adenosylhomocysteine, S-adenosylmethionine, S-amino-imadazole-4-carboxamide, asparagine, cadaverine, cystamine, citrulline, diaminopimelic acid, 2,4-diaminobutyric acid, cysteamine, glutamine,
3-hydroxykynurenine, kynurenine, putrescine and negamycin.

**10.** A method as claimed in claim 5, wherein the steroid moiety of the bile acid derivative is based on an acid selected from the group consisting of cholic acid, chenodeoxycholic acid, deoxycholic acid, hyodeoxycholic acid, hyocholic acid, α-, β- or ω-muricholic acid, nor-bile acids, lithocholic acid, 3β-hydroxycholenoic acid, ursodeoxycholic acid and allocholic acid (5α-cholan-24-oic-acid).

**11.** A method as claimed in claim 5, wherein the fluorescent bile acid derivative is a cholyl-lysyl-fluorescein (CLF) having the formula:-

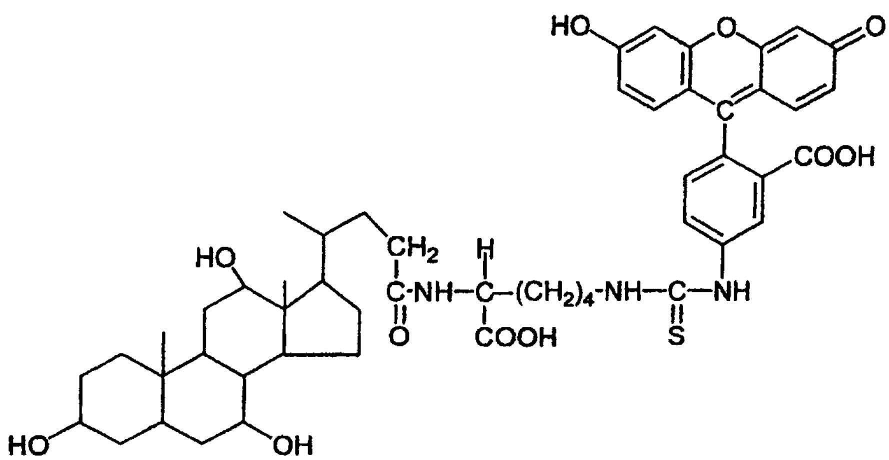

wherein the lysyl and fluorescein are linked by a thioamido moiety -C(S)NH-.

**12.** The use of a coloured or fluorescent bile acid derivative in the manufacture of a diagnostic agent for the determination of liver function by a method which comprises the steps of:-

(i) introducing an effective amount of the diagnostic agent containing the coloured or fluorescent bile acid derivative intravenously into a patient,
(ii) collecting samples of blood which has passed through the liver of the patient at timed intervals after step (i), and
(iii) assessing the colour or fluorescence of bile acid derivative in each sample by:-

(a) processing the blood sample to obtain blood plasma containing the bile acid derivative,
(b) measuring the colour or fluorescence of the bile acid derivative in the blood plasma, and
(c) fitting the measurements obtained in step (iii)(b) to a plasma elimination curve and comparing the plasma elimination curve with plasma elimination curves obtained from individuals having known liver function, to arrive at a determination of liver function for the patient.

**13.** An in-vitro method of determining the liver function of a patient, comprising the steps of:-

(i) processing a blood sample which has passed through the liver of the patient and which has been collected a predetermined time interval after introducing an effective amount of a coloured or fluorescent bile acid derivative intravenously into the patient; and
(ii) assessing the colour or fluorescence of bile acid derivative in the sample by:-

(a) processing the blood sample to obtain blood plasma containing the bile acid derivative,
(b) measuring the colour or fluorescence of the bile acid derivative in the blood plasma, and
(c) comparing the measurement obtained in step (b) with at least one standard so as to arrive at a determination of liver function for the patient.

## Patentansprüche

1. In vitro Verfahren zum Bestimmen der Leberfunktion eines Patienten, umfassend die Stufen von

(i) Verarbeiten einer Serie von Blutproben, die die Leber des Patienten passiert haben, und die zu geregelten Intervallen gesammelt worden sind nach Einführen einer wirksamen Menge eines gefärbten oder fluoreszierenden Gallensäurederivats intravenös in den Patienten, und
(ii) Untersuchen der Farbe oder Fluoreszenz von Gallensäurederivat in jeder Probe durch:

(a) Verarbeiten der Blutprobe unter Erhalten von Blutplasma, enthaltend das Gallensäurederivat,
(b) Messen der Farbe oder Fluoreszenz des Gallensäurederivats in dem Blutplasma und
(c) Anpassen der in Stufe (b) erhaltenen Messungen an eine Plasmaeliminierungskurve und Vergleichen der Plasmaeliminierungskurve mit Plasmaeliminierungskurven, erhalten von Individuen mit bekannter Leberfunktion, unter Gelangen zu einer Bestimmung von Leberfunktion für den Patienten.

2. Verfahren nach Anspruch 1, wobei Stufe (ii)(a) durch Zentrifugation der Probe erreicht wird.

3. Verfahren nach Anspruch 2, wobei ein fluoreszierendes Gallensäurederivat verwendet wird, und Stufe (ii)(a) umfaßt zusätzlich Trennung von Blutproteinen aus dem Plasma vor Zentrifugation.

4. Verfahren nach Anspruch 1, 2 oder 3, einschließend die zusätzliche Stufe von Verdünnen des Plasmas zwischen Stufen (ii)(a) und (ii)(b).

5. Verfahren nach einem vorhergehenden Anspruch, wobei das Gallensäurederivat umfaßt (a) einen Steroidrest mit (i) mindestens einem unblockierten Substituenten, ausgewählt aus der Gruppe, bestehend aus einem unblockierten 3-Hydroxylsubstituenten, einem unblockierten 7-Hydroxylsubstituenten und einem unblockierten 12-Hydroxylsubstituenten, und (ii) einer unblockierten Carboxylgruppe, angefügt durch eine Amidkopplung an eine Seitenkette des Steroidrestes, und (b) einen aktiven Rest, der an die Leber zu richten ist, wobei der aktive Rest an ein α-Kohlenstoffatom relativ zu der unblockierten Carboxylgruppe angefügt ist.

6. Verfahren nach einem von Ansprüchen 1 bis 4, wobei das Gallensäurederivat die allgemeine Formel (I) hat:

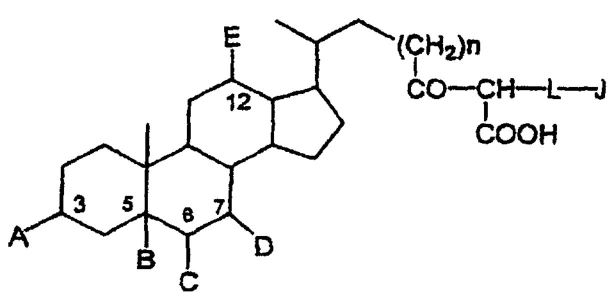

(I)

wobei A ausgewählt ist aus der Gruppe, bestehend aus α-OH und β-OH; B ist ausgewählt aus der Gruppe, bestehend aus α-H und β-H; C ist ausgewählt aus der Gruppe, bestehend aus -H, α-OH und β-OH; oder B und C zusammen bilden eine Doppelbindung; D ist ausgewählt aus der Gruppe, bestehend aus -H, α-OH und β-OH; E ist ausgewählt aus der Gruppe, bestehend aus -H, α-OH und β-OH; L ist ein Kopplungsrest; J ist der gefärbte oder fluoreszierende Rest; und n ist 0 oder 1.

7. Verfahren nach Anspruch 6, wobei J ein Fluorescein, Rhodamin oder anderer fluoreszierender Rest ist oder einschließt.

8. Verfahren nach Anspruch 6 oder 7, wobei der Kopplungsrest N-terminal an seinem Ende, angefügt an aktiven Rest J, ist, und L ausgewählt ist aus der Gruppe, bestehend aus

$$-(CH_2)_nNH,$$

wobei n 3 oder 4 ist,

$$-(CH_2)_4NH-(CH_2)_3NHC(=NH)NH-$$

und

$$-(CH_2)_2-CH(OH)CH_2NH-.$$

9. Verfahren nach Anspruch 6, wobei der Rest -NH-CH(COOH)-L- in der allgemeinen Formel (I) von einer Verbindung abstammt, ausgewählt aus der Gruppe, bestehend aus S-Adenosylhomocystein, S-Adenosylmethionin, S-Aminoimidazol-4-carboxamid, Asparagin, Cadaverin, Cystamin, Citrullin, Diaminopimelinsäure, 2,4-Diaminobuttersäure, Cysteamin, Glutamin, 3-Hydroxykynurenin, Kynurenin, Putrescin und Negamycin.

10. Verfahren nach Anspruch 5, wobei der Steroidrest des Gallensäurederivats auf einer Säure basiert, ausgewählt aus der Gruppe, bestehend aus Cholsäure, Chenodeoxycholsäure, Deoxycholsäure, Hyodeoxycholsäure, Hyocholsäure, α-, β- oder ω-Muricholsäure, Nor-Gallensäuren, Lithocholsäure, 3β-Hydroxycholensäure, Ursodeoxycholsäure und Allocholsäure (5α-Cholan-24-oic-Säure).

11. Verfahren nach Anspruch 5, wobei das fluoreszierende Gallensäurederivat ein Cholyl-lysyl-fluorescein (CLF) mit der Formel ist:

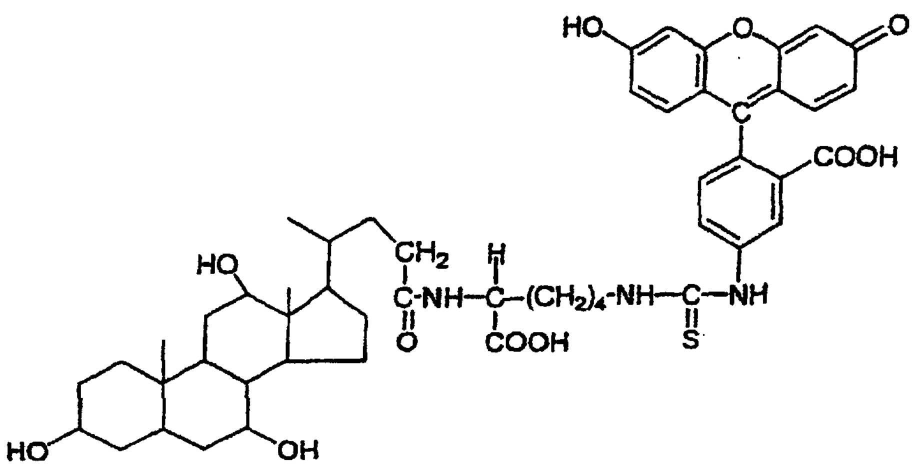

wobei das Lysyl und Fluorescein durch einen Thioamidrest -C(S)NH- gekoppelt sind.

12. Verwendung eines gefärbten oder fluoreszierenden Gallensäurederivats bei der Herstellung eines diagnostischen Mittels für die Bestimmung von Leberfunktion durch ein Verfahren, das die Stufen umfaßt von:

(i) Einführen einer wirksamen Menge des diagnostischen Mittels, enthaltend das gefärbte oder fluoreszierende Gallensäuredrivat, intravenös in einen Patienten,
(ii) Sammeln von Blutproben, die die Leber des Patienten passiert haben, zu geregelten Intervallen nach Stufe

(i) und
(iii) Untersuchen der Farbe oder Fluoreszenz von Gallensäurederivat in jeder Probe durch:

(a) Verarbeiten der Blutprobe unter Erhalten von Blutplasma, enthaltend das Gallensäurederivat,
(b) Messen der Farbe oder Fluoreszenz des Gallensäurederivats in dem Blutplasma und
(c) Anpassen der in Stufe (iii)(b) erhaltenen Messungen an eine Plasmaeliminierungskurve und Vergleichen der Plasmaeliminierungskurve mit Plasmaeliminierungskurven, erhalten von Individuen mit bekannter Leberfunktion unter Gelangen zu einer Bestimmung von Leberfunktion für den Patienten.

**13.** In vitro Verfahren zum Bestimmen der Leberfünktion eines Patienten, umfassend die Stufen von:

(i) Verarbeiten einer Blutprobe, die die Leber des Patienten passiert hat, und die zu einem vorher bestimmten Zeitintervall gesammelt worden ist nach Einführen einer wirksamen Menge eines gefärbten oder fluoreszierenden Gallensäurederivats intravenös in den Patienten, und
(ii) Untersuchen der Farbe oder Fluoreszenz von Gallensäurederivat in der Probe durch:

(a) Verarbeiten der Blutprobe unter Erhalten von Blutplasma, enthaltend das Gallensäurederivat,
(b) Messen der Farbe oder Fluoreszenz des Gallensäurederivats in dem Blutplasma und
(c) Vergleichen der in Stufe (b) erhaltenen Messung mit mindestens einem Standard, um so zu einer Bestimmung von Leberfunktion für den Patienten zu gelangen.

**Revendications**

**1.** Procédé *in vitro* de détermination de la fonction hépatique d'un patient, comprenant les étapes consistant

(i) à traiter une série d'échantillons de sang qui sont passés à travers le foie du patient et qui ont été recueillis à des intervalles temporisés après avoir introduit une quantité efficace d'un dérivé d'acide biliaire coloré ou fluorescent par voie intraveineuse dans le patient; et
(ii) à évaluer la couleur ou la fluorescence du dérivé d'acide biliaire dans chaque échantillon:-

(a) en traitant l'échantillon de sang pour obtenir le plasma sanguin contenant le dérivé d'acide biliaire,
(b) en mesurant la couleur ou la fluorescence du dérivé d'acide biliaire dans le plasma sanguin, et
(c) en adaptant les mesures obtenues dans l'étape (b) à une courbe d'élimination de plasma et en comparant la courbe d'élimination de plasma avec les courbes d'élimination de plasma obtenues à partir de personnes ayant une fonction hépatique connue, pour arriver à une détermination de la fonction hépatique pour le patient.

**2.** Procédé selon la revendication 1, dans lequel l'étape (ii)(a) est effectuée par centrifugation de l'échantillon.

**3.** Procédé selon la revendication 2, dans lequel un dérivé d'acide biliaire fluorescent est employé, et l'étape (ii)(a) comprend de plus la séparation des protéines sanguines du plasma avant la centrifugation.

**4.** Procédé selon la revendication 1, 2 ou 3, incluant l'étape supplémentaire consistant à diluer le plasma entre les étapes (ii)(a) et (ii)(b).

**5.** Procédé selon l'une quelconque des revendications précédentes, dans lequel le dérivé d'acide biliaire comprend (a) une fraction stéroïdienne ayant (i) au moins un substituant non bloqué choisi dans le groupe constitué par un substituant 3-hydroxyle non bloqué, un substituant 7-hydroxyle non bloqué et un substituant 12-hydroxyle non bloqué, et (ii) un groupe carboxyle non bloqué attaché au moyen d'une liaison amide à une chaîne latérale de la fraction stéroïdienne; et (b) une fraction active qui doit être ciblée au foie, ladite fraction active étant attachée à un atome de carbone en $\alpha$ par rapport au groupe carboxyle non bloqué.

**6.** Procédé selon l'une quelconque des revendications 1 à 4, dans lequel le dérivé d'acide biliaire a la formule générale (I):-

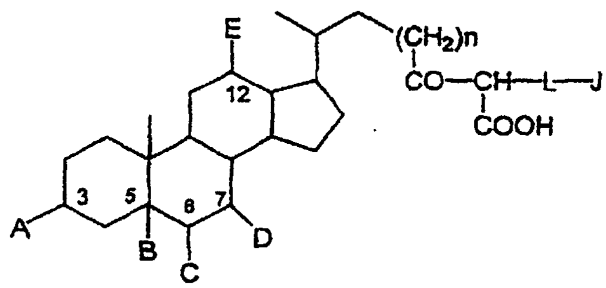

(I)

dans laquelle A est choisi dans le groupe constitué par α-OH et β-OH; B est choisi dans le groupe consitué par α-H et β-H; C est choisi dans le groupe constitué par -H, α-OH et β-OH; ou B et C forment ensemble une liaison double; D est choisi dans le groupe constitué par -H, α-OH et β-OH; E est choisi dans le groupe constitué par -H, α-OH et β-OH; L est une fraction de liaison; J est ladite fraction colorée ou fluorescente; et n est 0 ou 1.

**7.** Procédé selon la revendication 6, dans lequel J est ou inclut une fluorescéine, de la rhodamine ou une autre fraction fluorescente.

**8.** Procédé selon la revendication 6 ou 7, dans lequel la fraction de liaison est terminée par N à son extrémité attachée à la fraction active J, et L est choisi dans le groupe constitué par:

$$-(CH_2)_nNH,$$

où n est 3 ou 4,

$$-(CH_2)_4NH-(CH_2)_3NHC(=NH)NH-,$$

et

$$-(CH_2)_2-CH(OH)CH_2NH-.$$

**9.** Procédé selon la revendication 6, dans lequel la fraction -NH-CH(COOH)-L- dans la formule générale (I) est obtenue à partir d'un composé choisi dans le groupe constitué par la S-adénosylhomocystéine, la S-adénosylméthionine, le S-amino-imidazole-4-carboxamide, l'asparagine, la cadavérine, la cystamine, la citrulline, l'acide diaminopimélique, l'acide 2,4-diaminobutyrique, la cystéamine, la glutamine, la 3-hydroxycynurénine, la cynurénine, la putrescine et la négamycine.

**10.** Procédé selon la revendication 5, dans lequel la fraction stéroïdienne du dérivé d'acide biliaire est à base d'un acide choisi dans le groupe constitué par l'acide cholique, l'acide chénodésoxycholique, l'acide désoxycholique, l'acide hyodésoxycholique, l'acide hyocholique, l'acide α-, β- ou ω-muricholique, les acides nor-biliaires, l'acide lithocholique, l'acide 3β-hydroxycholénoïque, l'acide ursodésoxycholique et l'acide allocholique (acide 5α-cholan-24-oïque).

**11.** Procédé selon la revendication 5, dans lequel le dérivé d'acide biliaire fluorescent est une cholyl-lysyl-fluorescéine (CLF) ayant la formule:-

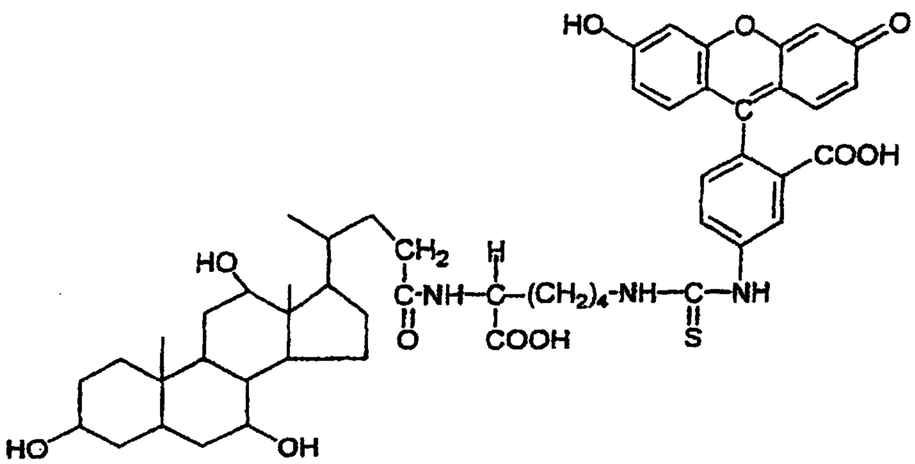

dans laquelle le groupe lysyle et la fluorescéine sont liés par une fraction thioamido -C(S)NH-.

**12.** Utilisation d'un dérivé d'acide biliaire coloré ou fluorescent dans la fabrication d'un agent diagnostique pour déterminer la fonction hépatique par un procédé qui comprend les étapes consistant:-

(i) à introduire une quantité efficace de l'agent diagnostique contenant le dérivé d'acide biliaire coloré ou fluorescent par voie intraveineuse dans un patient,

(ii) à recueillir des échantillons de sang qui est passé à travers le foie du patient à des intervalles temporisés après l'étape (i), et

(iii) à évaluer la couleur ou la fluorescence du dérivé d'acide biliaire dans chaque échantillon:-

(a) en traitant l'échantillon de sang pour obtenir du plasma sanguin contenant le dérivé d'acide biliaire,

(b) en mesurant la couleur ou la fluorescence du dérivé d'acide biliaire dans le plasma sanguin, et

(c) en adaptant les mesures obtenues dans l'étape (iii)(b) à une courbe d'élimination de plasma et en comparant la courbe d'élimination de plasma avec les courbes d'élimination de plasma obtenues à partir de personnes ayant une fonction hépatique connue, pour arriver à une détermination de la fonction hépatique pour le patient.

**13.** Procédé *in vitro* de détermination de la fonction hépatique d'un patient, comprenant les étapes consistant:-

(i) à traiter un échantillon de sang qui est passé à travers le foie du patient et qui a été recueilli à un intervalle de temps prédéterminé après avoir introduit une quantité efficace d'un dérivé d'acide biliaire coloré ou fluorescent par voie intraveineuse dans le patient; et
(ii) à évaluer la couleur ou la fluorescence du dérivé d'acide biliaire dans l'échantillon:

(a) en traitant l'échantillon de sang pour obtenir du plasma sanguin contenant le dérivé d'acide biliaire,

(b) en mesurant la couleur ou la fluorescence du dérivé d'acide biliaire dans le plasma sanguin, et

(c) en comparant la mesure obtenue dans l'étape (b) avec au moins un étalon de façon à arriver à une détermination de la fonction hépatique pour le patient.

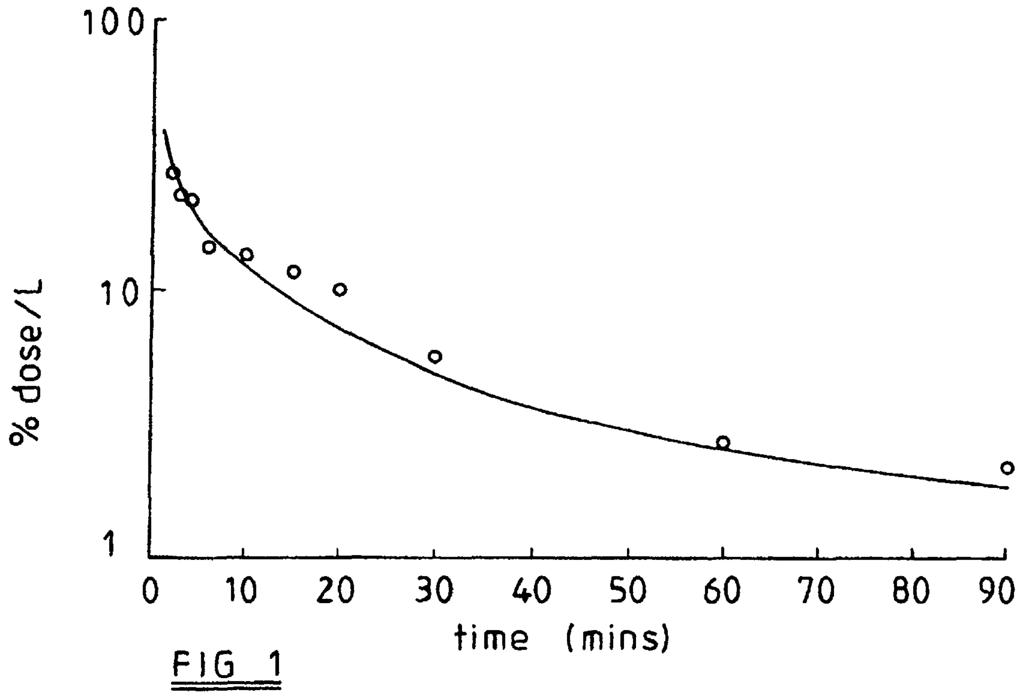
100
10
1
% dose/L
0
10
20
30
40
50
60
70
80
90
time (mins)

FIG 1

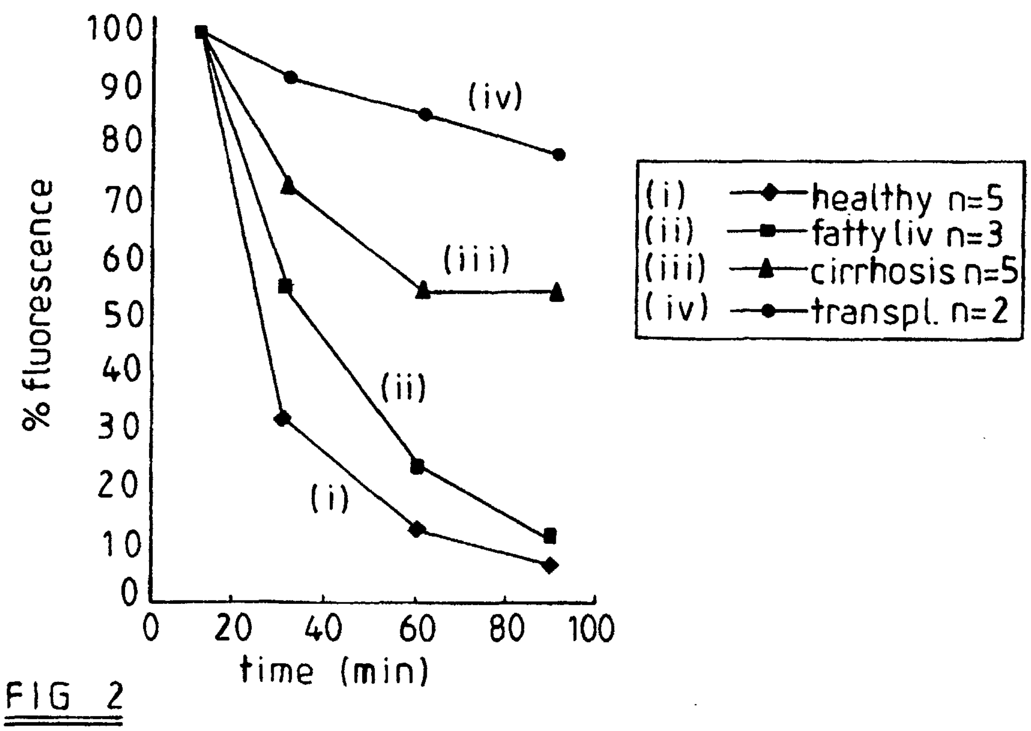
100
90
80
70
60
50
40
30
20
10
0
% fluorescence
(iv)
(iii)
(ii)
(i)
0
20
40
60
80
100
time (min)
(i) healthy n=5
(ii) fatty liv n=3
(iii) cirrhosis n=5
(iv) transpl. n=2

FIG 2